# EUROPEAN PATENT APPLICATION

(11) **EP 1 132 406 A1**
(43) Date of publication of application: **12.09.2001**
(21) Application number: 99958191.1
(22) Date of filing: 24.11.1999
(51) Int. Cl.: C07K 14/815, C12N 15/15

(54) **INHIBITOR OF METALOCARBOXYPEPTIDASES AS FIBRINOLYTIC AGENT**

(30) Priority: 25.11.1998 ES 9802524
(71) Applicant: UNIVERSITAT AUTONOMA DE BARCELONA, 08193 Bellaterra (ES); Ludwig Maximilians Universität München, 80336 München (DE)
(72) Inventor: REVERTER, David, 08193 Bellaterra (ES); VENDRELL, Josep, 08193 Bellaterra (ES); CANALS, Francesc, 08193 Bellaterra (ES); HORSTMANN, Jeanny, 08193 Bellaterra (DE); QUEROL, Enrique, 08193 Bellaterra (ES); FRITZ, Hans, 80336 Munich (DE); SOMMERHOFF, Christian, P., 80336 Munich (DE); AVILES, Francesc, X., 08193 Bellaterra (ES)
(74) Representative: Ponti Sales, Adelaida
(86) International application number: ES9900378
(87) International publication number: WO0031140

(57) **Abstract**

The invention refers to the gene of a carboxymetallocarboxipeptidase inhibitor from the medical leech *Hirudo medicinalis,* the sequence of the same and the protein that it codifies, the characterization of the same. The invention also refers to its pharmacological application as fibrinolytic agent.

## Description

### FIELD OF THE INVENTION

The present invention refers to metallocarboxypeptidase inhibitors as fibrinolytic agents. In particular, the present invention refers to the identification, cloning and sequence determination of the gene and the protein that it encodes and to the application of the fibrinolytic properties of one of them from the medical leech *Hirudo medicinalis,* the LCI (Leech Carboxypeptidase Inhibitor).

### 1.- BACKGROUND OF THE INVENTION

Proteases and their inhibitors are involved in many biological processes, such as coagulation and fibrinolysis (Fritz, H., Schmidt, I., and Turk, V. (eds) (1990) Special volume on Proteinase Inhibitors and Biological Control, Biol. Chem. Hoppe-Seyler, Vol. 371; Avilés, F. X. (ed) (1993) Innovations in Proteases and Their Inhibitors, Walter de Gruyter, Berlin), forming the metallocarboxypeptidase subfamily forms an important group within the proteases (Hooper, N. M. (ed) (1996) Zinc Metalloproteases in Health and Disease, Taylor and Francis Ltd., London). Unlike endopeptidase inhibitors, only a few metallocarboxypeptidase inhibitors have been identified (Avilés et al. (1993) Eur. J. Biochem. 211, 381-389; Hass & Ryan (1981) Methods Enzymol. 80, 778-791; Homandberg et al. (1989) Arch. Biochem. Biophys. 270, 153-161; Normant et al. (1995) Proc. Natl. Acad. Sci. U. S. A. 92, 12225-12229). The inhibitors from Solanacea and Ascaris inhibit carboxypeptidases through their C-terminal tail, that interacts with the target enzyme in a substrate-like manner. On the contrary, the inhibitor from rat brain inhibits them through a region that shows sequence similarity to the inhibitory loop in the "pro" regions of these enzymes, that is positioning the inhibitory loop on the active site cleft of the enzymes (Coil et al. (1991) EMBO J. 10, 1-9; Guasch et al. (1992) J. Mol. Biol. 224, 141-57; García-Saez et al. (1997) EMBO J. 16, 6906-6913).

A variety of proteinaceous protease inhibitors have been isolated from leeches (Ascenzi et al. (1995) Mol. Aspects Med. 16, 215-313). Among them, some act as anticoagulants, such as the thrombin-specific hirudins and the factor Xa-specific antistasin (Tuszynsky et al. (1987) J. Biol. Chem. 262, 9718-9723). It has to be emphasized that the medical leech seem to have inhibitors directed against all proteases of human mast cells (tryptase, chymase, cathepsin G and metallocarboxypeptidase A). When activated in the infective processes, mast cells release enzymes that contribute to initiate the host defense mechanisms. The inhibitors produced by leeches could have the function of blocking the mentioned host defense mechanisms [Huntley et al. (1990) Parasite Immunol. 12, 85-95; Douch et al. (1996) Int. J. Parasitol. 26, 91-95; Miller, H. R. (1996) Vet. Immunol. Immunopathol. 54, 331-336; Arizono et al. (1996) Clin. Exp. Immunol. 106, 55-61].

Here, it is described the gene sequence, the protein that it encodes and some characteristics of a new metallocarboxypeptidase inhibitor obtained from medical leech, the first one described for this organism, that has been named LCI (Leech Carboxypeptidase Inhibitor). Its recombinant production and biological activity tests are also described. The LCI shows very little sequence similarity with other carboxypeptidase inhibitors previously described. The LCI may take part in the elimination of blood clots by inhibiting plasma carboxypeptidase B (or metallocarboxypeptidase B), also known as TAFI, an enzyme recently shown to retard fibrinolysis (Bajzar et al. (1995) J. Biol. Chem. 270, 14477-14484; Sakharov et al. (1997) J. Biol. Chem. 272, 14477-14482).

### 2.- SUMMARY OF THE INVENTION

It is an objective of the present invention the identification of a metallocarboxypeptidase inhibitor, named LCI (Leech Carboxypeptidase Inhibitor), from the medical leech *Hirudo medicinalis.*

Another objective of the present invention is the determination of the sequence of the gene and of the protein that it encodes, and the characterization of the latter as a molecule with a high metallocarboxypeptidase inhibitory activity.

A further aim of the present invention is the use of the metallocarboxypeptidase inhibitor, according with sequence ID No. 2, alone or combined with other fibrinolytic agents which it enhances or complements, to prepare a drug useful as fibrinolytic agent.

The present invention also refers to a pharmaceutical composition that comprises, as active agent, an effective quantity of such metallocarboxypeptidase inhibitor, or its derivatives, and a pharmaceutically acceptable excipient.

Finally, it is an aim of the present invention the characterization of the fibrinolytic activity of the metallocarboxypeptidase inhibitor, the LCI, as its main therapeutical and applied purpose.

### 3.- DETAILED DESCRIPTION OF THE INVENTION

The aims of the present invention are related to the identification, cloning and sequence determination of the gene and the protein that it encodes, and to its fibrinolytic properties, of a metallocarboxypeptidase inhibitor from the medical leech *Hirudo medicinalis,* named LCI (Leech Carboxypeptidase Inhibitor).
In a preferred embodiment of the present invention, the proposed objectives have been achieved through a process which includes the partial objectives described next.
First, a metallocarboxypeptidase inhibitory activity has been isolated and identified in extracts from the medical leech *Hirudo medicinalis* (LCI from Leech Carboxypeptidase Inhibitor). Also, a purification procedure of the LCI, either native or recombinant, and procedures for its characterization have been established.

In further embodiment of the present invention it has been obtained a peptide sequence of the N-terminal fragment of the native LCI inhibitor, said inhibitor being obtained and purified from extracts of the leech *Hirudo medicinalis.* The information obtained from this sequence has been essential to design several oligonucleotides that allowed the LCI gene cloning.

In a further embodiment of the present invention a cDNA library has been prepared which, together with the oligonucleotides previously mentioned, allowed the design of a PCR-RACE strategy by which the LCI gene cloning has been achieved. A direct consequence of this embodiment has been the determination of the nucleotide sequence of the mentioned gene and the sequence of the protein that it encodes.

In a further embodiment of the present invention, systems for the heterologous expression of the recombinant LCI (rLCI), alone or as fusion protein, have been designed. Also, the corresponding protocols for the proteolytic separation of the rLCI from the fusion protein and the purification and characterization procedures of the rLCI have been designed.

In a further embodiment of the present invention, the fibrinolytic activity of the LCI has been determined, which is related with its metallocarboxypeptidase inhibitory activity. The therapeutical utility of the LCI is based on its mentioned fibrinolytic activity.

Finally, an example is described that ilustrates the preferred embodiment of the present invention which, however, is is not intended to include all the design and application possibilities of the present invention.

### 4.- EXAMPLE OF THE PREFERED EMBODIMENT OF THE PRESENT INVENTION

The aims of the present invention have been achieved through the identification, the sequence determination of the gene and the protein that it encodes and the determination of its fibrinolytic properties, of a metallocarboxypeptidase inhibitor from the medical leech *Hirudo medicinalis,* named LCI (Leech Carboxypeptidase Inhibitor).

### 4.A. Isolation, purification, mass determination and sequence analysis of the LCI.

LCI was purified from extracts of the medical leech *Hirudo medicinalis* (GEN Therapeutica, Bad Zwischenahn). Lyophilized leech extract (0.5 g) was dissolved in 20 mM Tris acetate (pH 8.0) buffer, centrifuged at 13,000 x g for 10 min. and, after pH equilibration, the supernatant was loaded onto a preparative anion-exchange column (TSK-DEAE 5PW, 2.5 x 15 cm; Toyo-Soda) connected to a FPLC system (Amersham Pharmacia Biotech), and elution was performed with a linear gradient from 0% to 100% 0.8 M ammonium acetate in 20 mM Tris acetate at a flow rate of 4 ml/min for 80 min. Fractions were collected and the metallocarboxypeptidase inhibitory activities of pancreatic CPA1, CPA2 and CPB were determined. The inhibitory activity of the LCI for such metallocarboxypeptidases, as well as for the plasma carboxypeptidase B (or metallocarboxypeptidase B), the most outstanding for the present invention, and the corresponding Ki were determined according to previously described tests (Burgos et al. (1989) J. Chromatog., 481, 233-243; Molina et al. (1992) Gene 116, 129-138 and (1994) J. Biol. Chem. 269, 21467-21472). The resulting Ki were ~0.4 nM at pH 7.5. Inhibitor-containing fractions (64 min and 69 min fractions) were lyophilized and subjected to reverse-phase HPLC (Vydac C4 column) using a linear gradient from 20% to 42% acetonitrile in 0.1% trifluoroacetic acid at a flow rate of 1 ml/min for 60 min. The metallocarboxypeptidase inhibitory activity eluted at 38 and 34 min of retention time. The purity of the isolated inhibitor was checked by SDS-Tricine gel electrophoresis (Schägger & Von Jagow (1986) Anal. Biochem. 166, 369-376). The molecular mass was analyzed using the MALDI-TOF (BRUKER) mass spectrometry technique. It was also performed the sequence analysis of the N-terminal and internal residues by Edman automatic degradation. The sequence of the mentioned 28 residues corresponds to that of the section "List of sequences".

### 4.B. Molecular cloning and sequence analysis of the LCI cDNA

Degenerated oligonucleotides were designed based on the N-terminal and an internal amino acid sequence of purified LCI, which allowed an amplification of cDNA by RACE-PCR (Fritz et al. (1991) Nucleic Acids Res. 19, 3747-3753; Frohman et al. (1988) Proc. Natl. Acad. Sci. U. S. A. 85, 8998-9002; Chenchik et al. (1996) BioTechniques 21, 526-534). The primers, synthesized by MWG-Biotech (Ebersberg, Germany), were as follows:
N1, 5'-GACGAATCNTTYYTNTGYTAYCA-3' with N = A, C, G, or T and Y = C or T (deduced from amino acids 5-12 of LCI)
N2, 5'-TGTGCTAYCARCCNGAYCARGT-3' with R = A or G (from amino acids 9-16)
N3, 5'-CCAGACCARGTNTGYTGYTTYAT-3' (from amino acids 13-20) C1, 5'-CCTGTGSWRTANGGNACCCA-3' with S = G or C and W = A or T (from amino acids 55-49)
YXT, 5'-CGAGGGGGATGGTCGACGGAAGCGACCT18-3' (modified from Fritz et al. (1991) Nucleic Acids Res. 19, 3747-3753) Y, 5'-CGAGGGGGATGGTCGACGG-3' (representing a part of YXT) X, 5'-GATGGTCGACGGAAGCGACC-3' (representing a part of YXT)

Total RNA was isolated from frozen leeches using the guanidinium thiocyanate procedure (Chomczynski & Sacchi (1987) Anal. Biochem. 162, 156-159), and poly(A)+ RNA was purified by oligo(dT) affinity separation (Amersham Pharmacia Biotech). First-strand cDNA synthesis (Amersham Pharmacia Biotech) was carried out with the oligonucleotide YXT as primer. First, an internal cDNA fragment and the 3' (3'-RACE) were amplified. In a first round of PCR, primers N1, N2, and N3 in all combinations with the gene-specific primer C1 or the adaptor primers X and Y were used. PCR amplifications were performed with the Goldstar polymerase (Eurogentec, Belgium), using 30 cycles each with 94 °C for 20 s, annealing at 53 °C for 1 min, and extension at 72 °C for 2 min. PCR products were separated by electrophoresis on 1.8% agarose/Tris acetate gels. PCR products generated by amplifications were eluted from the gel, subcloned using the pGEM-T AT-cloning system (Promega) and used to transform *Eschecichia coli* strain JM109. The 5' end of the LCI-cDNA was obtained by a 5'-RACE approach using the Marathon cDNA amplification kit (CLONTECH). This was done using the Marathon adaptor primer AP1 and the gene-specific primers 5'-TAGTCAAGAAGAGAAATGCCCT-3' and 5'-TTAGCCTCGCATCAGTGACACACG-3' (complementary to nucleotides 361-340 and 300-277 of the complete cDNA; see section "List of sequences). The sequence analysis of the different partial sequences amplifeid allowed the design of new primers for the 5'-RACE. Finally, the LCI cDNA sequence was obtained, which consisted in a 243 bp ORF, in addition to two not translated regions, one of 21 bp upstream to the ORF and one of 182 bp downstream to the ORF (see section "List of sequences"). The ORF translation generates a sequence of 81 aminoacides, that contain a signal peptide of 15 residues, which implies a mature LCI sequence of 66 aminoacides (see section "List of sequences"). A search in the sequence databases showed that no sequence similar to that of LCI had been described previously.

### 4.C. Heterologous expression of LCI

For the heterologous expression of LCI, the vectors pIN-III-OmpA3 (Ghrayeb et al. (1984) EMBO J. 3, 2437-2442; Molina et al. (1992) Gene 116, 129-138; Molina et al. (1994) J. Biol. Chem. 269, 21467-21472) and pET-32b (Promega) were used. pIN-III-OmpA3 was cleaved with EcoRI, blunt-ended with Mung bean nuclease (Boehringer Mannheim), and digested with BamHI. This linearized vector was ligated with the final product obtained (described in the previous section) to generate the plasmid pIN-III-OmpA3-LCI, which was used to transform E. coli strain MC1061. Similarily, pET-32b was cleaved with EcoRV and BamHI to generate a linearized vector with a blunt end and a BamHI end, and ligated with the PCR product to generate pET-32b-LCI. This plasmid was used to transform E. coli strain ADA494.
For the production of recombinant LCI (rLCI), 5 ml of MC1061/pIN-III-OmpA3-LCI inoculum were grown overnight at 37 °C in M9CAS (containing 0.3% glycerol) and used to inoculate 0.5 liter of the same medium. After growth for 2 h, IPTG was added to a final concentration of 0.5 mM. At 24 h after induction, the culture was centrifuged at 10,000 x g for 20 min, and the supernatant was applied to a Sep-Pak Plus C18 cartridge (Waters, Millipore). The bound material was eluted with 40 ml of 30% 2-propanol and concentrated in a Roto-Vapor to remove the organic solvent. Subsequently, recombinant LCI was purified essentially as described in section 4.A. The rLCI was in the culture medium. Quantification was performed by determining the metallocarboxypeptidase inhibitory activity.
In the case of ADA494/pET-32b-LCI plasmid, the procedure was as follows. 5 ml of ADA494/pET-32b-LCI were grown overnight in Luria-Bertani (LB) medium and used to inoculate 1 liter of the same medium. When the optical density of the culture reached values of 0.4-0.6, IPTG was added to a final concentration of 0.4 mM. At 3 h after induction, recombinant LCI produced intracellularly as a thioredoxin fusion protein was purified using a Ni2+ column (Amersham Pharmacia Biotech). The fusion protein eluted with 1 M imidazol, 0.5 M NaCl, 20 mM Tris/HCl, pH 7.9. Subsequently, the fusion protein was cleaved with enterokinase (Sigma), and the recombinant LCI purified as described in section 4.A. Quantification was performed by determining the metallocarboxypeptidase inhibitory activity.
In both cases, the expression was optimized. In the case of the pIN-III-OmpA3 system, the highest yield was achieved using 0.3% glycerol and 0.5 mM IPTG according with previous results in similar systems (Molina et al. (1992) Gene 116, 129-138). The yield, when cultured in flask, was 3.4 mg/l of supernatant, with a 60% of recovery. The recombinant LCI was purified as described in section 4.A., and as a control of the correct processing by the host cell, a sequence analysis of the N-terminal end and a determination of the molecular mass by MALDI-TOF mass spectrometry were performed. In the case of the pET-32b system, 20-40 mg/l of the fusion thioredoxin-rLCI were obtained. After enterokinase digestion, 7 mg/l were obtained, with a 50% recovery.

### 4.D. LCI fibrinolytic activity test

The LCI promotes the degradation of the fibrin clots by inhibiting the plasma carboxypeptidase B (or metallocarboxypeptidase B) or TAFI (the inhibition constant is ~0.4 nM). This is consistent with the fact that this enzyme inhibits the fibrinolysis by destroying the binding sites of plasminogen to fibrin (Sakharov et al. (1997) J. Biol. Chem. 272, 14477-14482). The test was performed with the purified fibrinolysis components. The initial coagulation (promoted by thrombin) and the subsequent fibrinolysis (induced by plasminogen activator) was monitored over time by the increase or decrease of the turbidity in a spectrophotometric test at 405 nm (Bajzar et al (1995) J. Biol. Chem. 271, 16603-16608). Different concentrations of plasma carboxypeptidase B (or metallocarboxypeptidase B), previously activated from its zymogen by a thrombin (20 nM) - thrombomodulin (50 nM) mixture, were tested against rising LCI concentrations in a buffer containing 0.02 M HEPES, 0.15 M NaCl, 5 mM CaCl₂, 0.01% Tween80, pH 7.4. The fibrinolysis medium consisted of fibrinogen (3.36 mM), Glu plasminogen (0.89 mM), α2-antiplasmin (0.56 mM) and antithrombin III (1.11 nM). This mixture was mixed with the different concentrations previously prepared of plasma carboxypeptidase B (or metallocarboxypeptidase B) - LCI mixtures and tested in a well microplate containing thrombin (6.0 nM) and plasminogen activator (441 pM). The turbidity at 405 nm of the samples of each microplate well was monitored every 2.5 min at 37°C, and the mean time of lysis of the clot was measured. In those samples that did not contain LCI, the time needed for the lysis of the clot was much higher (never reaching more than 5% of lysis in 30 hours). In contrast, in the presence of LCI, the plasma carboxypeptidase B (or metallocarboxypeptidase B) was inhibited and, then, the turbidity of the medium disappeared faster. The destruction of the clot resulted much faster (lysis was 75% in 1.5 hours, and reached 100% in less than 3 hours).

4.E. List of sequences
GENERAL INFORMATION
(i) APPLICANT
(A) NAME: UNIVERSITAT AUTÓNOMA DE BARCELONA
(B) STREET: Campus UNIVERSITARI
(C) CITY: BELLATERRA
(D) PROVINCE: BARCELONA
(E) COUNTRY: ESPAÑA
(F) POSTAL CODE (CP):08193
(G) TELEPHONE: 93-581-16 36

(A) NAME: LUDWIG MAXIMILIANS UNIVERSITÄT MÜNCHEN
(B) STREET: 20 NUSSBAUMSTRASSE
(C) CITY: MÜNCHEN
(D) COUNTRY: ALEMANIA
(E) POSTAL CODE (CD):D-80336
(ii) TITLE OF INVENTION: METALOCARBOXINPEPTIDASE INHIBITOR
AS FIBRINOLYTIC AGENT
(iii) NUMBER OF SEQUENCES: 2
(iv) COMPUTER READABLE FORM:
(A) MEDIUM TYPE: Floppy disk
(B) COMPUTER: IBM PC Compatible
(C) OPERATING SYSTEM: PC-DOS/MS-DOS
(D) SOFTWARE: Patentin Release #1.0, Version #1.30
(EPO)

SEQ ID NO:1
LOCUS: double stranded linear cDNA
DEFINITION: metalocarboxipeptidase inhibitor gene from
Hirudo medicinalis
CHARACTERISTICS: Location/Qualifiers
/product ="LCI"
/start codon=22
/stop codon = 265
ORIGINAL SOURCE: (a) Organism:Hirudo medicinalis

SEQUENCE DESCRIPTION 129 A 99 C 96 G 141 T
GACTTGGTAA CTCATTCGAT CATGTTTCTG CTCGTTTTCC TGTGCTGCCT 50
CCACCTGGTG ATTTCGTCGC ATACACCAGA TGAGAGTTTC TTGTGCTACC 100
AACCAGACCA GGTGTGCTGT TTCATTTGCA GAGGAGCGGC ACCTTTGCCT 150
TCAGAAGGGG AATGCAATCC ACATCCTACA GCACCCTGGT GCCGGGAAGG 200
GGCTGTAGAG TGGGTTCCCT ACTCTACTGG TCAATGTCGC ACAACCTGCA 250
TCCCATATGT CGAGTAGATG ACCCATCGTG TGTCACTGAT GCGAGGCTAA 300
CTCTCATTAT TTTCCTGAAC GCATCCTTGT TGAAATTTAA GGGCATTTCT 350
CTTCTTGACT AATTATTTTG CTGAGTTAAA ATAATAAAAT AATATTGAAG 400
CATTATTTAA TAATGTTCTC GTTTGAATAA AATATGATCG AAAGATAAAA 450
AAAAAAGAAA AAAAA
465

SEQ ID NO:2
1 gacttggtaactcattcgatc 21
22 ATG TTT CTG CTC GTT TTC CTG TGC TGC CTC CAC CTG GTG 60
-15 M F L L V F L C C L H L V -3

61 ATT TCG TCG CAT ACA CCA GAT GAG AGT TTC TTG TGC TAC 99
-2 I S S H T P D E S F L C Y 11

100 CAA CCA GAC CAG GTG TGC TGT TTC ATT TGC AGA GGA GCG 138
12 Q P D Q V C C F I C R G A 24

139 GCA CCT TTG CCT TCA GAA GGG GAA TGC AAT CCA CAT CCT 177
25 A P L P S E G E C N P H P 37

178 ACA GCA CCC TGG TGC CGG GAA GGG GCT GTA GAG TGG GTT 216
38 T A P W C R E G A V E W V 50

217 CCC TAC TCT ACT GGT CAA TGT CGC ACA ACC TGC ATC CCA 255
51 P Y S T G Q C R T T C I P 63

256 TAT GTC GAG tagatgacccatcgtgtgtcactgatgcgaggctaactc 303
64 Y V E * 66

304 tcattattttcctgaacgcatccttgttgaaatttaagggcatttctcttc 354
355 ttgactaattattttgctgagttaaaataataaaataatattgaagcatta 405
406 tttaataatgttctcgtttgaataaaatatgatcgaaagataaaaaaaaaa 456
457 gaaaaaaaa 465

## Claims

1. A recombinant nucleotide sequence that encodes a protein sequence corresponding to a metallocarboxypeptidase inhibitor from *Hirudo medicinalis*.

2. A nucleotide sequence according to claim 1, **characterized in that** it comprises the sequence identified as SEQ ID N° 1 of the list of sequences.

3. A polypeptide sequence encoded by the nucleotide sequence according to claims 1 and 2, **characterized in that** it comprises the sequence identified as SEQ ID N° 2 of the list of sequences.

4. A polypeptide sequence according to claim 3, wherein such sequence is substantially homologous to the sequence identified as SEQ N° 2.

5. A nucleotide sequence that comprises a coding sequence of a polypeptide substantially homologous to the sequence ID N° 2 according to claim 3.

6. A prokaryotic or eukaryotic expression vector **characterized in that** it includes the recombinant nucleotide sequence of any of claims 1, 2 or 5, and in that it is able to express the biologically active metallocarboxypeptidase inhibitor.

7. A transformed *Escherichia coli* cell **characterized in that** it comprises an expression vector according to claim 6 and in that it is able to produce the biologically active metallocarboxypeptidase inhibitor.

8. A procedure to prepare the recombinant metallocarboxypeptidase inhibitor according to any of claims 3 to 4 **characterized in that** it comprises
(i) the culture of the transformant that contains an expression vector capable of expressing a biologically active metallocarboxypeptidase inhibitor; and
(ii) its obtention and purification.

9. A procedure according to claim 8 **characterized in that** the recombinant process takes place in a prokaryotic or eukaryotic host.

10. A metallocarboxypeptidase inhibitor according to claims 3 or 4, as fibrinolytic agent.

11. Use of the metallocarboxypeptidase inhibitor according to claims 3 or 4, to prepare a drug useful as fibrinolytic agent.

12. Use of the metallocarboxypeptidase inhibitor according to claim 11, in combination with other fibrinolytic agents which it complements or enhances, to prepare a drug useful as fibrinolytic agent.

13. A pharmaceutical composition that comprises, as active agent, an effective quantity of the metallocarboxypeptidase inhibitor, or its derivatives, according to any of the previous claims, and a pharmaceutically acceptable excipient.
